(19) Europäisches Patentamt / European Patent Office / Office européen des brevets

(11) **EP 3 572 061 A1**

(12) **EUROPEAN PATENT APPLICATION**
published in accordance with Art. 153(4) EPC

(43) Date of publication:
**27.11.2019 Bulletin 2019/48**

(21) Application number: **17893313.1**

(22) Date of filing: **21.12.2017**

(51) Int Cl.:
**A61J 1/05** (2006.01)    **B65D 65/00** (2006.01)

(86) International application number:
**PCT/JP2017/045893**

(87) International publication number:
**WO 2018/135228 (26.07.2018 Gazette 2018/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **18.01.2017  JP 2017006657**

(71) Applicants:
• **Daikin Industries, Ltd.**
  **Osaka-shi, Osaka 530-8323 (JP)**
• **Otsuka Pharmaceutical Factory, Inc.**
  **Tokushima 772-8601 (JP)**

(72) Inventors:
• **KOMAZAWA, Kozue**
  **Osaka-shi**
  **Osaka 530-8323 (JP)**

• **HIGUCHI, Tatsuya**
  **Osaka-shi**
  **Osaka 530-8323 (JP)**
• **KIGAWA, Koji**
  **Osaka-shi**
  **Osaka 530-8323 (JP)**
• **OBATA, Kazuaki**
  **Osaka-shi**
  **Osaka 530-8323 (JP)**
• **DEMPO, Takayuki**
  **Naruto-shi**
  **Tokushima 772-8601 (JP)**
• **NISHIMURA, Masuhiro**
  **Naruto-shi**
  **Tokushima 772-8601 (JP)**

(74) Representative: **D Young & Co LLP**
  **120 Holborn**
  **London EC1N 2DY (GB)**

(54) **CONTAINER FOR ADMINISTRATION, STORAGE, DELIVERY OR TRANSPORTATION OF PROTEIN HAVING LOW PROTEIN ADSORBABILITY OR PROTEIN-CONTAINING COMPOSITION, AND APPARATUS FOR PRODUCING PROTEIN OR PROTEIN COMPOSITION**

(57)    There is provided a container or an equipment having low-protein adsorption properties, to be used for administering, storing, conveying or transporting protein or a composition including protein, or for producing protein or a composition including protein. A container or an equipment for producing protein or a composition including protein, wherein the surface of the container or the equipment in contact with protein or a composition including protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of non-fluorinated group terminals and -CF$_2$H group terminals in the fluororesin of 70 or less per $1 \times 10^6$ carbon atoms, has remarkable low-protein adsorption properties. Such a container or equipment can be used to thereby, for example, prevent, in a producing (culturing and purifying, and the like) step, a storing and delivering step and/or administration of a protein formulation such as an antibody pharmaceutical product, the protein formulation from being lost due to adsorption thereof to the equipment.

**Description**

**Technical Field**

The present invention relates to a container to be used for administering, storing, conveying or transporting protein or a composition including protein, and an equipment for producing protein or a protein composition, wherein the surface of the container or the equipment in contact with protein or a composition including a protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of non-fluorinated group terminals and -$CF_2$H group terminals in the fluororesin of 70 or less per $1 \times 10^6$ carbon atoms.

**Background Art**

Proteins themselves and compositions including proteins are frequently treated in the research fields of medicine, pharmacy, agriculture, biology and the like, or in the technical field of production of protein formulations such as pharmaceutical products, especially, antibody pharmaceutical products, and in the technical field of regenerative medicine.

Proteins play an essential and important role in preserving life, for example, transferring information and producing and/or conveying physiologically active substances in vivo. When proteins and compositions including proteins are used in the above research fields and technical fields, however, a large problem due to adsorption of protein is often caused. For example, in a producing (culturing and purifying, and the like) step and a storing and delivering step of a protein formulation such as an antibody drug, adsorption of the protein formulation to an equipment causes loss, thereby resulting in an increase in production cost. Further, in administration of a protein formulation such as an antibody drug, adsorption of the protein formulation to an equipment can cause loss, thereby resulting in a decrease in an actual amount of administration as compared with the amount of administration described in a container, to thereby cause the therapeutic effect to be affected. Further, in a cell culturing step for the purposes of regenerative medicine, studies of cells, and the like, a problem is that adsorption of an expensive protein ingredient (for example, a growth factor necessary for cell growth, differentiation induction or the like) contained in a culture medium (particularly, a serum-free culture medium or a culture medium for differentiation induction) to an equipment during culturing causes loss, thereby leading to an increase in cost. Furthermore, irreversible adsorption of protein causes a chromatographic column and/or a tube for experimental use to be fouled, and also causes activation of a complement system in a blood-permeable membrane or the like and remarkably deteriorates the original membrane permeability, thereby not allowing functions such as substance exchange to be sufficiently exerted. Additionally, cell activation and immunoreaction are induced, and materials are immediately recognized as foreign substances.

In recent years, protein formulations such as antibody drugs have been increasingly important in the technical field of pharmaceutical products. On the other hand, regenerative medicine utilizing various cells, tissues, and the like including iPS cells and cell sheets has also been progressively put to practical use. Accordingly, a material which essentially weakly interacts with protein and which allows no protein to absorb thereto is desired in many fields.

Various materials low in adsorption properties to protein and various medical and experimental tools where such materials are used (container, syringe, catheter, laboratory tool, therapeutic device, and the like) have been heretofore proposed. For example, the following non-fluorinated polymers for the purpose of prevention of adsorption of biologically related substances such as protein have been proposed: an ethylene-vinyl alcohol copolymer (Patent Document 1), a polyurea-urethane polymer (Patent Document 2), a mixture of a water-soluble copolymer and a hydrazide compound having at least two hydrazino groups per molecule (Patent Document 3), a copolymer made of a plurality of repeating units (Patent Documents 4 and 5), a hydrophilized oil or hydrophilized copolymer (Patent Document 6), a blend of a water-soluble polymer and a matrix polymer (Patent Document 7), a copolymer of 2-methacryloyloxyethyl phosphorylcholine with n-butyl (meth)acrylate, methyl (meth)acrylate or styrene, wherein the copolymer is soluble in a buffer and saline (Patent Document 8), a copolymer including a repeating unit derived from (a) an ethylene-based unsaturated polymerizable monomer having an alkylene glycol residue and (b) an ethylene-based unsaturated polymerizable monomer where a functional group immobilizing a physiologically active substance is bound via an alkylene glycol residue, wherein the copolymer has a reactive functional group at least one terminal of the copolymer (Patent Document 9), and a cyclic olefin resin (Patent Document 10).

Further, in a case where a fluororesin is used as the material low in adsorption properties to protein, the followings have been proposed, as a compound and a coating liquid for prevention of protein attachment, capable of forming a covering layer which is excellent in water resistance, from which a covering ingredient is hardly eluted, to which protein hardly adsorbs and which is excellent in biocompatibility, as well as a medical device where the compound for prevention of protein attachment is used: a compound for prevention of protein attachment, for forming a covering layer preventing protein adsorption on the surface of an article, wherein the compound includes a fluorine-containing polymer, as well as a medical device having on the surface thereof a covering layer formed from the compound for prevention of protein attachment (Patent Document 11). Patent Document 11 describes the following: "the fluorine atom content of the fluorine-containing polymer is preferably 5 to 90% by mass, more preferably 10 to 85% by mass, especially preferably 15 to 80% by mass; when the fluorine atom content is equal to or more than the lower limit, water resistance is excellent, and when the fluorine atom content is equal to or less than the upper limit, protein hardly adsorbs" (paragraph [0013]). In Patent Document 11, a large number of fluorine-containing resins including FEP and PFA are exemplified, and there is described in Examples formation of a film layer on a well surface by use of a coating liquid obtained by dissolving FEP having a fluorine atom content of 76.0% in chloroform.

**Prior Art Documents**

**Patent Documents**

Patent Document 1: Japanese unexamined Patent Application Publication No. 01-213137
Patent Document 2: Japanese unexamined Patent Application Publication No. 05-103831
Patent Document 3: Japanese Patent No. 4941672
Patent Document 4: Japanese Patent No. 5003902
Patent Document 5: Japanese Patent No. 5207012
Patent Document 6: Japanese unexamined Patent Application Publication (Translation of PCT Application) No. 2002-505177
Patent Document 7: Japanese unexamined Patent Application Publication (Translation of PCT Application) No. 07-502563
Patent Document 8: Japanese Patent No. 3443891
Patent Document 9: Japanese unexamined Patent Application Publication No. 2008-1794
Patent Document 10: Japanese unexamined Patent Application Publication No. 2016-155327
Patent Document 11: Japanese unexamined Patent Application Publication No. 2016-26520

**Summary of the Invention**

**Object to be Solved by the Invention**

An equipment produced using each of the non-fluorinated polymer materials described in Patent Documents 1 to 10 above is said to be insufficient in durability and oil resistance, and cannot be said to perfectly exert the predetermined effect of low-protein adsorption properties in terms of practical use.

Further, an equipment produced using a fluorinated base material described in Patent Document 11 above has the problem of being insufficient in low-protein adsorption properties even in the case of use of FEP.

An object of the present invention is to provide a container for administering, storing, conveying or transporting protein or a composition including protein, and an equipment for producing protein or a protein composition, which can solve the problems of the prior arts and can sufficiently exert low-protein adsorption properties.

**Means to Solve the Object**

As a result of exhaustive studies to solve the problem, the present inventors have found that when a container wherein the surface of the container in contact with protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of non-fluorinated group terminals and -CF$_2$H group terminals in the fluororesin of 70 or less per $1 \times 10^6$ carbon atoms is used to administer protein or a composition including protein, store protein or a composition including protein in the container, or convey or transport it, or when parts for producing a protein composition, wherein the surface of the member in contact with protein is formed, is used to produce a protein composition, protein, or a composition including protein, such as a protein formulation, is effectively inhibited from adsorbing to the inner surfaces of the container and the equipment for producing, and protein, or a composition including protein, such as a protein formulation, is remarkably prevented from being lost due to adsorption thereof to the container and the equipment for producing. This finding has led to the completion of the present invention.

That is, the present invention is as follows.

(1) A container for administering, storing, conveying or transporting protein or a composition including protein, or an equipment for producing protein or a composition including protein, wherein the surface of the container or the equipment in contact with protein or a composition including protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of a non-fluorinated group terminal and a -$CF_2H$ group terminal in the fluororesin of 70 or less per $1 \times 10^6$ carbon atoms.

(2) The container for administering, storing, conveying or transporting protein or a composition including protein, or the equipment for producing protein or a composition including protein according to (1), which is a container.

(3) The container for administering, storing, conveying or transporting protein or a composition including protein, or the equipment for producing protein or a composition including protein according to (1) or (2), which is a bag.

(4) The container for administering, storing, conveying or transporting protein or a composition including protein, or the equipment for producing protein or a composition including protein according to any one of (1) to (3), wherein the protein or the composition including protein is an antibody (immunoglobulin).

(5) The container for administering, storing, conveying or transporting protein or a composition including protein, or the equipment for producing protein or a composition including protein according to any one of (1) to (3), wherein the protein or the composition including protein is albumin.

(6) The container for administering, storing, conveying or transporting protein or a composition including protein, or the equipment for producing protein or a composition including protein according to (1), which is an equipment for producing a protein formulation.

**Effect of the Invention**

The fluororesin in the present invention, especially FEP·PFA having a total number of non-fluorinated group terminals and -$CF_2H$ group terminals of 70 or less per $1 \times 10^6$ carbon atoms, exhibits remarkable low-protein adsorption properties, and therefore, when the container for administering, storing, conveying or transporting, wherein the surface of the container in contact with protein or a composition including protein is formed of such a polymer, according to the present invention, is used to administer and/or store protein or a composition including protein, convey or transport protein or a composition including protein, or the equipment for producing protein or a composition including protein, wherein the surface of the equipment in contact with protein or a composition including protein is formed of such a polymer, according to the present invention, is produced, the following advantages are achieved.

(1) In a producing (culturing and purifying, and the like) step, a storing and delivering step and/or administration of a protein formulation such as an antibody pharmaceutical product, the protein formulation is prevented from being lost due to adsorption thereof to the equipment.

(2) In a cell culturing step (including a differentiation induction step) in a regenerative medicine application and the like, expensive protein ingredients (for example, growth factors necessary for cell proliferation, differentiation induction and the like, specifically, various proteins (for example, cell proliferation factors such as albumin, insulin and transferrin, and cytokines and growth factors such as activin A, a bone morphogenetic factor 4 (BMP-4), an epithelial cell growth factor (EGF), a stem cell factor (SCF) and interleukins)) contained in a culture medium (particularly, a serum-free culture medium or a culture medium for differentiation induction) are prevented from being lost due to adsorption thereof to the equipment during culturing (leading to a decrease in cost).

**Mode of Carrying Out the Invention**

The container for administering, storing, conveying or transporting protein or a composition including protein, or the equipment for producing protein or a composition including protein according to the present invention (hereinafter, referred to simply as "the container or the equipment according to the present invention" or "the container or the equipment" in some cases) is not especially limited as long as it is a container or an equipment to be used for administering, storing, conveying or transporting protein or a composition including protein, or to be used for producing protein or a composition including protein, wherein the surface of the container or the equipment in contact with protein or a composition including protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of non-fluorinated group terminals and -$CF_2H$ group terminals in the fluororesin of 70 or less per $1 \times 10^6$ carbon atoms (hereinafter, these fluororesins are generically referred to as "the present fluororesin" in some cases); and the container or the equipment as a whole is optionally formed of the present fluororesin. The container or the equipment according to the present invention has a feature in that the container or equipment surface in contact with protein or a composition including protein is formed of the present fluororesin. A container or an equipment having such a feature can be used to administer, store, convey or transport protein (for example, antibody (immunoglobulin)) or a composition including protein, thereby, for example, preventing, in a producing (culturing and purifying, and the like) step, a storing and delivering step and/or administration of a protein formulation such as an antibody pharmaceutical product, the protein formulation from being lost due to adsorption thereof to the equipment, and expensive protein ingredients (for example, growth factors and cytokines necessary for cell growth, differentiation induction and the like) from being lost due to adsorption thereof to the equipment, leading to a decrease in cost.

In the present invention, the "protein" means a single polymer compound or multiple polymer compounds made by linearly linking (polymerizing) many L-amino acids via amide bonds (also referred to as "peptide bonds"), and is not limited with respect to the number of amino acids serving as constituent elements. Accordingly, so-called peptide is also encompassed in the protein in the present invention. Further, glycoprotein obtained by binding sugar and protein, and lipoprotein obtained by binding lipid and protein are also encompassed in the protein in the present invention. Examples of the protein to be used in the present invention include albumin, fibrinogen, globulin ($\alpha$1-globulin, $\alpha$2-globulin, $\beta$-globulin, $\gamma$-globulin), erythropoietin, collagen, elastin, keratin, lactoferrin, avidin, cadherin, proteoglycan, mucin, LDL (Low Density Lipoprotein), HDL (High Density Lipoprotein), VLDL (Very Low Density Lipoprotein), a cell proliferation factor such as insulin and transferrin, and cytokine and a growth factor such as activin A, a bone morphogenetic factor 4 (BMP-4), an epithelial cell growth factor (EGF), a stem cell factor (SCF) and interleukin, but are not limited thereto.

The composition including protein in the present invention means a mixture or a product of one or more proteins and one or more substances other than such proteins. Examples of the composition including protein, to be used in the present invention, include a protein formulation such as an antibody pharmaceutical product, a body fluid such as blood, a biological ingredient including protein such as serum and plasma, and a culture medium including a protein ingredient (particularly, a serum-free culture medium and a culture medium for differentiation induction), but are not limited thereto.

With respect to the container for administering, storing, conveying or transporting protein or a composition including protein in the present invention, the "container for administering", "container for storing", "container for conveying", "container for transporting", and "equipment for producing" and "equipment" mean the followings, respectively.

The "container for administering" means a container to be used in the case of administering protein or a composition including protein to a patient in a clinical setting.

The "container for storing" means a container to be used in the case of storing protein or a composition including protein for a certain period.

The "container for conveying" means a container to be used in the case of moving protein or a composition including protein by a human power, a machine (including a robot), or the like.

The "container for transporting" means a container to be used in the case of carrying protein or a composition including protein by transport means such as a car, a ship, and an airplane.

The "equipment for producing" means an equipment to be used in the case of producing protein or a composition including protein.

The "equipment" means a material for making tools (simple utensils), instruments (relatively compact and small-scale devices and utensils (instruments) to be directly operated by human beings), and tools and instruments. Examples include a pipe, a tube and a container of production facility for antibody pharmaceutical products, etc., and a purification equipment (for example, a filter and a column).

In the present fluororesin, the total number of non-fluorinated group terminals (for example, functional groups such as -COF, -COOH, -COOH associated with water, - CH$_2$OH, -CONH$_2$ and -COOCH$_3$) and -CF$_2$H group terminals in the fluororesin is preferably 70 or less per $1 \times 10^6$ carbon atoms, more preferably 35 or less per $1 \times 10^6$ carbon atoms. Furthermore, the total number is more preferably 20 or less per $1 \times 10^6$ carbon atoms, especially preferably 10 or less per $1 \times 10^6$ carbon atoms. Any fluororesin including no -CF$_2$H group terminal is optionally adopted, and when no -CF$_2$H group terminal is included, the number of non-fluorinated group terminals in the fluororesin is preferably 70 or less per $1 \times 10^6$ carbon atoms, more preferably 35 or less per $1 \times 10^6$ carbon atoms. Further, the number is still more preferably 20 or less per $1 \times 10^6$ carbon atoms, and especially preferably 10 or less per $1 \times 10^6$ carbon atoms.

The number per $1 \times 10^6$ carbon atoms of the -COF, - COOH, -COOH associated with water, -CH$_2$OH, -CONH$_2$, -COOCH$_3$ and -CF$_2$H can be calculated by FT-IR.

In the present invention, the "non-fluorinated group terminal" means a terminal having some reactivity and usually called an unstable terminal, and specifically includes functional groups such as -COF, -COOH, -COOH associated with water, -CH$_2$OH, -CONH$_2$ and -COOCH$_3$.

The melting point of the present fluororesin is 320°C or less, and is 240°C or more. Examples of a preferable range of the melting point include the range of 245°C or more and 315°C or less, and the range of 250°C or more and 310°C or less.

Specific examples of the present fluororesin include a tetrafluoroethylene (TFE)-hexafluoropropylene (HFP)-based copolymer (FEP) and a TFE-perfluoroalkylvinyl ether (PAVE)-based copolymer (PFA).

Among them, FEP·PFA having a total number of non-fluorinated group terminals and -CF$_2$H group terminals of 70 or less per $1 \times 10^6$ carbon atoms exhibits remarkable low-protein adsorption properties over FEP·PFA having a total number of non-fluorinated group terminals and -CF$_2$H group terminals of more than 70 per $1 \times 10^6$ carbon atoms. That is, while FEP·PFA having a total number of non-fluorinated group terminals and -CF$_2$H group terminals of more than 70 per $1 \times 10^6$ carbon atoms does not have sufficient low-protein adsorption properties, FEP·PFA having a total number of non-fluorinated group terminals and -CF$_2$H group terminals of 70 or less per $1 \times 10^6$ carbon atoms exhibits remarkably more excellent low-protein adsorption properties than FEP·PFA having a total number of non-fluorinated group terminals and -CF$_2$H group terminals of more than 70 per $1 \times 10^6$ carbon atoms. These properties are exhibited by only FEP·PFA having a total number of non-fluorinated group terminals and -CF$_2$H group terminals of 70 or less per $1 \times 10^6$ carbon atoms.

The fluororesin in the present invention has the following properties (1) to (5) other than the above properties.

(1) No elution of a plasticizer or the like.
(2) Possibility of high-temperature vapor (autoclave) sterilization.
(3) Insolubility in DMSO and DMF.
(4) Excellent cryogenic properties (no embrittlement even at -200°C).
(5) High transparency.

The "TFE-HFP-based copolymer" means a copolymer containing at least TFE and HFP. That is, the "TFE-HFP-based copolymer" includes, in addition to a binary copolymer (TFE/HFP copolymer; FEP) of TFE and HFP, also a ternary copolymer such as a copolymer of TFE, HFP and vinyl fluoride (VF) (TFE/HFP/VF copolymer), a copolymer of TFE, HFP and vinylidene fluoride (VDF) (TFE/HFP/VDF copolymer) and a copolymer of TFE, HFP and a perfluoro(alkylvinyl ether)(PAVE) (TFE/HFP/PAVE copolymer), a quaternary copolymer such as a copolymer of TFE, HFP, VF and VDF (TFE/HFP/VF/VDF copolymer), a copolymer of TFE, HFP, VF and PAVE (TFE/HFP/VF/PAVE copolymer) and a copolymer of TFE, HFP, VDF and PAVE (TFE/HFP/VDF/PAVE copolymer), and a quinary copolymer such as a copolymer of TFE, HFP, VF, VDF and PAVE (TFE/HFP/VF/VDF/PAVE copolymer).

The melting point of the present fluororesin, especially FEP, is 300°C or less, and is 240°C or more. Examples of a preferable range of the melting point include the range of 245°C or more and 290°C or less and the range of 250°C or more and 280°C or less.

The TFE-HFP-based copolymer is preferably a TFE/HFP copolymer or a TFE/HFP/PAVE copolymer. The mass ratio of TFE and HFP in such a TFE/HFP copolymer is preferably 80 to 97 / 3 to 20, and more preferably 84 to 92 / 8 to 16. Further, the mass ratio of TFE, HFP and PAVE in the TFE/HFP/PAVE copolymer is preferably 70 to 97 / 3 to 20 / 0.1 to 10, and more preferably 81 to 92 / 5 to 16 / 0.3 to 5.

The "TFE-PAVE-based copolymer" means a copolymer containing at least TFE and PAVE. That is, the "TFE-PAVE-based copolymer" includes, in addition to a binary copolymer of TFE and PAVE (TFE/PAVE copolymer; PFA), also a ternary copolymer such as a copolymer of TFE, PAVE and hexafluoropropylene (HFP) (TFE/PAVE/HFP copolymer), a copolymer of TFE, PAVE and vinylidene fluoride (VDF) (TFE/PAVE/VDF copolymer) and a copolymer of TFE, PAVE and chlorotrifluoroethylene (CTFE) (TFE/PAVE/CTFE copolymer), a quaternary copolymer such as a copolymer of TFE, PAVE, HFP and VDF (TFE/PAVE/HFP/VDF copolymer), a copolymer of TFE, PAVE, HFP and CTFE (TFE/PAVE/HFP/CTFE copolymer) and a copolymer of TFE, PAVE, VDF and CTFE (TFE/PAVE/VDF/CTFE copolymer), and a quinary copolymer such as a copolymer of TFE, PAVE, HFP, VDF and CTFE (TFE/PAVE/HFP/VDF/CTFE copolymer).

A PAVE constituting the PAVE unit is not especially limited, and examples thereof include perfluoro(methyl vinyl ether) [PMVE], perfluoro(ethyl vinyl ether) [PEVE], perfluoro(propyl vinyl ether) [PPVE], perfluoro(butyl vinyl ether), perfluoro(pentyl vinyl ether), perfluoro(hexyl vinyl ether) and perfluoro(heptyl vinyl ether).

The melting point of the present fluororesin, especially PFA, is 320°C or less, and is 285°C or more. Examples of a preferable range of the melting point include the range of 290°C or more and 315°C or less, the range of 295°C or more and 315°C or less, and the range of 300°C or more and 310°C or less.

The mass ratio of TFE and PAVE in the TFE-PAVE-based copolymer is preferably 90 to 98 / 2 to 10, and more preferably 92 to 97 / 3 to 8.

The present fluororesin can be fabricated by subjecting terminal groups of a fluororesin synthesized according to a usual method of suspension polymerization, emulsion polymerization or the like to a fluorination treatment by a known method such as a method in which before a fluororesin is melt extruded, the fluororesin and a fluorine-containing compound (for example, a fluorine radical source) are contacted with each other to carry out a stabilization treatment, and a method in which pellets of a fluororesin obtained after the fluororesin is melt extruded and a fluorine-containing compound are contacted with each other to carry out a fluorination treatment. Further, the present fluororesin can also be obtained by using a chain transfer agent and a polymerization catalyst capable of controlling terminal groups together with a fluorine monomer in production (polymerization reaction) of the fluororesin. Further, as the present fluororesin, commercially available products can be used. The fluorination treatment can also be carried out by contacting a fluorine-containing compound with moldings molded from fluororesins, like films molded by melting fluororesins, containers or equipments molded from the films and containers or equipments molded from fluororesins. Further, these treatment methods can also be combined.

That is, it is not needed that the total number of the non-fluorinated group terminals and the total number of the non-fluorinated group terminals and -CF$_2$H group terminals are 70 or less per $1 \times 10^6$ carbon atoms in each stage of fluororesins, pellets and films to become raw materials, and it suffices if on the surface of a final container or equipment in contact with protein, the total number thereof is 70 or less per $1 \times 10^6$ carbon atoms. Further, in the case of a fluororesin having one or more - CF$_3$ terminal groups, it is not needed that the number of the -CF$_3$ terminal groups is one or more in each stage of fluororesins, pellets and films to become raw materials, and it suffices if on the surface of a final container or equipment in contact with protein, the fluororesin has one or more -CF$_3$ terminal groups.

The fluorine radical source is not especially limited, but examples thereof include a halogen fluoride such as IF$_5$ and ClF$_3$, F$_2$ gas, CoF$_3$, AgF$_2$, UF$_6$, OF$_2$, N$_2$F$_2$ and CF$_3$OF. The F$_2$ gas is optionally of a concentration of 100%, but, from the safety aspect, is used by being mixed and diluted to 5 to 50% by mass, preferably 15 to 30% by mass, with an inert gas. The inert gas includes nitrogen gas, helium gas and argon gas, and from the viewpoint of the cost efficiency, nitrogen gas is preferable.

The fluorination treatment is carried out at a temperature of preferably 20 to 220°C, and more preferably 100 to 200°C. The fluorination treatment is carried out preferably for 5 to 30 hours, and more preferably for 10 to 20 hours.

The container or the equipment obtained by the present invention is optionally one in which the arithmetic mean roughness (Ra) of the surface roughness, the root-mean-square roughness (RMS) of the surface roughness, and the surface free energy have been regulated. Examples of the container or the equipment include one having its container inner surface or its equipment inner surface having an Ra of the surface roughness of 3.5 to 6.5 nm, an RMS of the surface roughness of 4.5 to 8.0 nm, and a surface free energy of 16.5 to 18.5 (mJ/m$^2$).

FEP·PFA having a total number of non-fluorinated group terminals and -CF$_2$H group terminals of 70 or less per $1 \times 10^6$ carbon atoms has extremely excellent properties as compared with FEP·PFA having a total number of non-fluorinated group terminals and -CF$_2$H group terminals of more than 70 per $1 \times 10^6$ carbon atoms, as described above, and especially has the following advantages.

(1) In a producing (culturing and purifying, and the like) step, a storing and delivering step and/or administration of a protein formulation such as an antibody pharmaceutical product, the protein formulation is prevented from being lost due to adsorption thereof to the equipment.

(2) In a cell culturing step (including a differentiation induction step) in a regenerative medicine application and the like, expensive protein ingredients (growth factors and cytokines necessary for cell proliferation, differentiation induction and the like) contained in a culture medium (particularly, a serum-free culture medium or a culture medium for differentiation induction) are prevented from being lost due to adsorption thereof to the equipment during culturing (leading to a decrease in cost).

The fluororesin in the present invention can be used for various containers having a surface in contact with protein or a composition including protein, and equipments such as a member of production facility, a purification equipment and an experimental tool.

Examples of forms of the container or the equipment according to the present invention include a bag, a bottle, a centrifugal tube, a vial, a syringe and a tube; in the case where the container according to the present invention is a container for administering protein, preferable are a syringe, a bag (for drip), a bottle (for drip) and a tube; in the case where the container according to the present invention is a container for storing protein, preferable are a bag, a bottle, a centrifugal tube and a vial; and in the case where the container according to the present invention is a container for conveying and transporting protein, preferable are a bag, a bottle, a vial and a tube. Particularly, the container according to the present invention having a bag shape, since it can be applied to every application for administering, storing, conveying and transporting protein, can be suitably exemplified. Examples of specific applications of the equipment for producing according to the present invention include the following.

(1) A relevant application for a protein formulation such as an antibody pharmaceutical product:
a culturing container (bag and the like), a pipe of production facility, a reaction or storage tank, etc., a purification equipment (filter, column and the like), a container for storing and delivering, a container for administering (syringe, administering bag and the like)
(2) A relevant application for culturing cells including a protein ingredient in a regenerative medicine application and the like:
a culturing container (bag and the like) (particularly, for culturing iPS cells in large amounts and for differentiation induction), a container for a culture medium (also including a container for a protein ingredient such as proliferation and growth factors, and cytokine)

The bag, bottle, centrifugal tube, vial, syringe, tube and the like can be produced by molding methods including compression molding, extrusion, transfer molding, inflation molding, blow molding, injection molding, rotation molding, lining molding, foam extrusion and film molding, as required in combination with sealing means such as heat sealing, high-frequency fusion and ultrasonic fusion. The case of production by these methods has the advantage of saving the trouble of coating as compared with the case of coating with a coating agent.

The bag can be produced specifically by overlapping films (sheets) of the present fluororesin material and heat sealing edge portions by using an impulse sealer.

The film to be used for molding of the bag may be a single-layer film or a laminated film consisting of a multilayer of two or more layers; and in the case of the laminated film consisting of a multilayer, it suffices if the bag is so molded that at least the inner surface in contact with mammal cells is a layer film of the present fluororesin material, and the other layer films are each allowed to be even a layer film of a material (for example, a polyolefinic resin material) different from the present fluororesin. The lamination of the films is carried out by using a method such as a heat lamination method, a heat compression method, a high-frequency heating method, a solvent casting method and an extrusion lamination method.

Further, the container or equipment according to the present invention can also be obtained by subjecting a base material, such as a bag, a bottle, a centrifugal tube, a vial, a syringe and a tube, produced of a glass, a metal, a resin or the like, to a coating treatment with a coating agent consisting of the present fluororesin. Any methods can be employed according to the form of the base material. Such a coating treatment includes spin coating, spray coating, bar coating, roll coating, dipping, brush coating, rotolining and electrostatic coating. The base material is coated with the fluororesin coating agent, and thereafter subjected to a drying treatment and a high-temperature heating treatment to thereby form a coating layer. Further, the coating layer is allowed to be made to be thick to any thickness by further double-coating a coating agent containing the present fluororesin.

Hereinafter, the present invention will be described more specifically by way of Examples, but the technical scope of the present invention is not limited to these Examples.

**Example 1**

1. Production of container

Each of 5 kinds of films of 10 cm $\times$ 4 cm in size and 100 $\mu$m in thickness was stacked for two films, and heat sealed by using an impulse sealer under the conditions of a sealing time of 50 sec, a sealing pressure of 0.2 MPa and a sealing width of 4 mm to thereby produce each of 5 kinds of perfluoropolymer bags (containers A to E).

Here, a polyethylene bag (container F) used was a commercially available bag of 70 $\times$ 50 $\times$ 0.04 mm in size (Unipac(R) A-4 manufactured by Seisannipponsha Ltd), and a glass container (container G) used was a commercially available screw pipe bottle having a size of $\varphi$ 21 mm in barrel diameter $\times$ 45 mm in total length (TS Screw tube bottle (9 mL) manufactured by Maruemu Corporation).

2. Measurement of the number of non-fluorinated group terminals and the number of -CF$_2$H terminals

Samples of corresponding resins of about 250 to 300 $\mu$m in thickness were fabricated, and analyzed by using an FT-IR Spectrometer 1760X (manufactured by PerkinElmer, Inc.).

The samples of corresponding resins of about 250 to 300 $\mu$m in thickness were fabricated, and subjected to measurement by using the films (fabricated from the pellets by melt molding) constituting the bags as they were, and in the case where the thickness was insufficient, by overlapping the films.

Difference spectra from standard samples (samples fluorinated enough until substantially no difference was any more observed in spectra) were acquired; absorbances of each peak were read; and the number of non-fluorinated group terminals and the number of CF$_2$H terminals per 1 $\times$ 10$^6$ carbon atoms were calculated for each sample according to the following equation. The number of non-fluorinated group terminals and the number of CF$_2$H terminals in each of the bags are shown in Table 2.

$$\text{The numbers of non-fluorinated group terminals and } -CF_2H$$
$$\text{terminals (per } 1 \times 10^6 \text{ carbon atoms)} = l \cdot k/t$$

l:    absorbance
k:    correction factor (see Table 1)
t:    sample thickness (mm)

[Table 1]

Table 1: Absorption Wave Number and Correction Factor of each Non-Fluorinated Group Terminal Group and -CF$_2$H Terminal

| Terminal Group | Absorption Wave Number (cm$^{-1}$) | Correction Factor |
|---|---|---|
| COF | 1884 | 405 |
| COOH(free) | 1813 | 455 |
| COOH(bonded) | 1775 1790 | 455 |
| COOCH$_3$ | 1795 | 355 |
| CONH$_2$ CH$_2$OH | 3438 3648 | 480 2325 |
| CF$_2$H | 3006 | 26485 |

[Table 2]

Table 2: The Number of Non-Fluorinated Group Terminals and the Number of -CF$_2$H Group Terminals in the Container

| Container Name | Material of Container | The Number of Non-Fluorinated Group Terminals | The Number of -CF$_2$H Terminals |
|---|---|---|---|
| A | FEP | 21 | 424 |
| B | FEP | 13 | 0 |
| C | FEP | 68 | 0 |
| D | PFA | 201 | 159 |
| E | PFA | 25 | 0 |
| F | Polyethylene | - | - |
| G | Glass | - | - |

**Example 2**

(Non-adhesiveness to protein)

(1) Preparation of coloring liquid and protein solution

The coloring liquid used was obtained by mixing 50 mL of a peroxidase coloring liquid (3,3',5,5'-tetramethylbenzidine (TMBZ), manufactured by KPL) and 50 mL of TMB Peroxidase Substrate (manufactured by KPL).
The protein solution used was obtained by diluting protein (POD-goat anti mouse IgG, manufactured by Bio-Rad Laboratories, Inc.) with a phosphate buffer solution (D-PBS, manufactured by Wako Pure Chemical Industries, Ltd.) by 16,000-fold.

(2) Protein adsorption

To each of containers A to G was dispensed 2 mL of the protein solution by a micropipette (used at 2 mL with respect to each of the containers), and left to stand at room temperature for 1 hour. Each reaction was performed at N = 3.

(3) Washing of container

Then, the protein solution was drained off from each of the containers, and thereafter each of the containers was washed with 4 mL of a phosphate buffer solution including 0.05% by mass of a surfactant (Tween 20, manufactured by Wako Pure Chemical Industries, Ltd.) four times (used at 4 mL with respect to each of the containers four times).

(4) Dispensing of coloring liquid

Then, 2 mL of the coloring liquid was dispensed to each of the containers washed (used at 2 mL with respect to each of the containers), and a coloring reaction was performed for 7 min. The coloring reaction was terminated by addition of 1 mL of a 1 M phosphoric acid solution (used at 1 mL with respect to each of the containers).

In a blank, 2 mL of the coloring liquid was dispensed to each of 3 glass containers (used at 2 mL with respect to each of the glass containers), and thereafter 40 μL of the protein solution was dispensed thereto. A coloring reaction was performed for 7 min, and terminated by addition of 1 mL of a 1 M phosphoric acid solution (used at 1 mL with respect to each of the glass containers).

(5) Preparation of absorbance measurement

Then, 3 mL of the liquid was taken out from each of the containers, and transferred to a cell for spectrophotometer.

(6) Absorbance measurement and protein adsorption rate Q

The absorbance was measured as the absorbance at 450 nm by an ultraviolet spectrophotometer U-3310 (manufactured by Hitachi Ltd.). Here, the average of the absorbance (N = 3) of the blank was designated as A0. The absorbance of the liquid moved from each of the bags was designated as A1.

The protein adsorption rate Q1 was determined according to the following equation, and the protein adsorption rate Q was determined as the average.

$$Q1 = A1/\{A0 \times (2000/\text{Amount of dispensing of protein solution of blank})\} \times 100$$

$$= A1/\{A0 \times (2000/40)\} \times 100 \ [\%]$$

[Table 3]

|  | Container Name | Protein Adsorption Rate Q (%) | Liquid Contact Area (cm$^2$) | Protein Adsorption Rate/cm$^2$ (%) |
|---|---|---|---|---|
| Comparative Example 1 | A | 1.12 | 13.2 | 0.085 |
| Example 1 | B | 0.24 | 13.2 | 0.018 |
| Example 2 | C | 0.46 | 13.2 | 0.035 |
| Comparative Example 2 | D | 1.03 | 13.2 | 0.078 |
| Example 3 | E | 0.14 | 13.2 | 0.011 |
| Comparative Example 3 | F | 2.10 | 12.0 | 0.175 |
| Comparative Example 4 | G | 0.95 | 9.4 | 0.101 |

As shown in Table 3, it was revealed that protein remarkably hardly adsorbed to the surface of each of containers B, C and E each using a perfluoropolymer having a total number of non-fluorinated group terminals and -CF$_2$H group terminals of 70 or less per $1 \times 10^6$ carbon atoms as compared with not only the surfaces of container F made of polyethylene and glass container G, but also the surfaces of containers A and D each using a perfluoropolymer having a total number of non-fluorinated group terminals and -CF$_2$H group terminals of more than 70 per $1 \times 10^6$ carbon atoms. In other words, the low-protein adsorption properties exhibited by a perfluoropolymer having a total number of non-fluorinated group terminals and -CF$_2$H group terminals of more than 70 per 1 $\times$ 10$^6$ carbon atoms were about 1/7 to about 1/2 those exhibited by a perfluoropolymer having a total number of non-fluorinated group terminals and -CF$_2$H group terminals of more than 70 per 1 $\times$ 10$^6$ carbon atoms.

**Example 3**

1. Production of container H

There was used, as container H, cover glass (manufactured by Matsunami Glass Ind., Ltd., C025251, 25 $\times$ 25 ·No.1) where a frame border of 10 $\times$ 10 mm was written on the surface by "PAP Pen Super-Liquid Blocker" (manufactured by Daido Sangyo Co., Ltd.).

2. Preparation of fluorescently labelled BSA solution

Fluorescently (Alexa Fluor(R) 555) labelled BSA was prepared by using commercially available BSA (bovine serum albumin, A7638 manufactured by Sigma-Aldrich) and a fluorescent labeling kit (Alexa Fluor(R) 555 NHS Ester (Succinimidyl Ester) A20009) manufactured by Thermo Fisher Scientific Inc., according to the protocol attached to the kit, and the fluorescently labelled BSA was diluted with PBS and adjusted so as to have a concentration of 10 $\mu$g/ mL, and the resulting fluorescently labelled BSA solution was used in the following experiment.

3. Adsorption of fluorescently labelled BSA

To each of containers A and B was dispensed 1 mL of the fluorescently labelled BSA solution (10 $\mu$g/mL) by a micropipette, and left to stand at 37°C for 1 hour. The liquid contact area to the bag was about 600 mm$^2$.

With respect to container H, the fluorescently labelled BSA solution was dropped by a micropipette into the frame border prepared by "PAP Pen Super-Liquid Blocker" so as to have a concentration of 167 $\mu$L/ cm$^2$, and placed in a petri dish and thereafter left to stand at 37°C for 1 hour. Each reaction was performed at N = 3.

4. Washing

After 1 hour, the fluorescently labelled BSA solution was removed from each of containers A, B and H, and thereafter each of the containers was washed with 2 mL of a PBS solution four times.

5. Measurement of fluorescence intensity

With respect to containers A and B, a part (square of about 10 $\times$ 10 mm) in contact with the fluorescently labelled protein solution was cut out by scissors, ProLong(R) Diamond antifade mountant (manufactured by Thermo Fisher Scientific Inc.) was dropped thereon, and thereafter cover glass was then mounted thereon, and the resultant was imaged by a fluorescence microscope (LSM700 manufactured by Zeiss, $\times$20).

Also with respect to container H, similarly, ProLong(R) Diamond antifade mountant (manufactured by Thermo Fisher Scientific Inc.) was dropped thereon after washing, new cover glass was mounted thereon, and the resultant was imaged by a fluorescence microscope (LSM700 manufactured by Zeiss, $\times$20).

Main imaging conditions are as follows;

- Objective lens: Plan-Apochromat 20X/0.8 M27
- Pinhole: 147 $\mu$m
- Number of pixels: 1024 $\times$ 1024
- Laser power: 0.5%

After imaging, analysis was made by Fiji software, thereby calculating the average fluorescence intensity derived from the fluorescently labelled BSA adsorbed, with respect to each sample. (analyzed at 5 fields of view with respect to each sample)

The proportion of the average fluorescence intensity (relative adsorption rate (%) of BSA) of each of containers A and B under the assumption that the average fluorescence intensity of container H is 100 is shown in Table 4.

[Table 4]

|  | Container Name | Relative Adsorption Rate of BSA (%) |
|---|---|---|
| Example 4 | B | 1.45 |
| Comparative Example 5 | A | 54.03 |
| Comparative Example 6 | H | 100 |

As shown in Table 4, it was revealed that BSA remarkably hardly adsorbed to the surface of container B using a perfluoropolymer having a total number of non-fluorinated group terminals and -CF$_2$H group terminals of 70 or less per 1 × 10$^6$ carbon atoms as compared with not only the surface of glass container H, but also the surface of container A using a perfluoropolymer having a total number of non-fluorinated group terminals and -CF$_2$H group terminals of more than 70 per 1 × 10$^6$ carbon atoms.

## Industrial Applicability

The fluororesin in the present invention exhibits extremely excellent low-protein adsorption properties, and therefore can be used in any equipment where protein or a composition including protein is used. Particularly, the fluororesin in the present invention can be utilized in various equipments associated with a protein formulation such as an antibody pharmaceutical product, for example, a culturing container (bag and the like), a pipe of production facility, etc., a purification equipment (filter, column and the like), a container for storing and delivering, a container for administering (syringe, administering bag and the like), and various equipments for producing associated with culturing of cells including a protein ingredient in a regenerative medicine application and the like, for example a culturing container (bag and the like) (particularly, for culturing iPS cells in large amounts and for differentiation induction) and a culture medium container (also including a container for a protein ingredient such as a growth factor).

## Claims

1. A container for administering, storing, conveying or transporting a protein or a composition including the protein, or an equipment for producing a protein or a composition including the protein, wherein a surface of the container or the equipment in contact with the protein or the composition including the protein is formed of a fluororesin which is at least one fluororesin selected from a tetrafluoroethylene-hexafluoropropylene-based copolymer and a tetrafluoroethylene-perfluoroalkylvinyl ether-based copolymer, which has a melting point of 320°C or less and which has a total number of a non-fluorinated group terminal and a -CF$_2$H group terminal in the fluororesin of 70 or less per 1 × 10$^6$ carbon atoms.

2. The container for administering, storing, conveying or transporting a protein or a composition including the protein, or the equipment for producing a protein or a composition including the protein according to claim 1, which is a container.

3. The container for administering, storing, conveying or transporting a protein or a composition including the protein, or the equipment for producing a protein or a composition including the protein according to claim 1 or 2, which is a bag.

4. The container for administering, storing, conveying or transporting a protein or a composition including the protein, or the equipment for producing a protein or a composition including the protein according to any one of claims 1 to 3, wherein the protein or the composition including the protein is an antibody (immunoglobulin).

5. The container for administering, storing, conveying or transporting a protein or a composition including the protein, or the equipment for producing a protein or a composition including the protein according to any one of claims 1 to 3, wherein the protein or the composition including the protein is albumin.

6. The container for administering, storing, conveying or transporting a protein or a composition including the protein, or the equipment for producing a protein or a composition including the protein according to claim 1, which is an equipment for producing a protein formulation.

## INTERNATIONAL SEARCH REPORT

| | International application No. |
|---|---|
| | PCT/JP2017/045893 |

### A.   CLASSIFICATION OF SUBJECT MATTER
Int. Cl.  A61J1/05(2006.01)i, B65D65/00(2006.01)i

According to International Patent Classification (IPC) or to both national classification and IPC

### B.   FIELDS SEARCHED

Minimum documentation searched (classification system followed by classification symbols)
Int. Cl.  A61J1/05, B65D65/00

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)

### C.   DOCUMENTS CONSIDERED TO BE RELEVANT

| Category* | Citation of document, with indication, where appropriate, of the relevant passages | Relevant to claim No. |
|---|---|---|
| X | JP 2008-539044 A (E. I. DU PONT DE NEMOURS AND COMPANY) 13 November 2008, paragraphs [0017]-[0022] & US 2006/0246244 A1, paragraphs [0016]-[0021] & WO 2006/119053 A1 & EP 1874915 A1 & CN 101166818 A | 1-6 |
| A | JP 2010-539252 A (DAIKIN INDUSTRIES, LTD.) 16 December 2010, paragraphs [0023], [0043], [0056], US 2011/0272173 A1, paragraphs [0031], [0062]-[0063], [0087] & WO 2009/044753 A1 & EP 2185648 A1 & ES 2628059 T3 | 1-6 |

☒   Further documents are listed in the continuation of Box C.     ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| | |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2017/045893 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | WO 2016/104596 A1 (NARA INSTITUTE OF SCIENCE AND TECHNOLOGY) 30 June 2016, paragraphs [0038]-[0116] (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1213137 A
- JP 5103831 A
- JP 4941672 B
- JP 5003902 B
- JP 5207012 B
- JP 2002505177 W
- JP 7502563 W
- JP 3443891 B
- JP 2008001794 A
- JP 2016155327 A
- JP 2016026520 A